# FASCICULE DE BREVET EUROPEEN

(11) **EP 0 648 513 B1**
(45) Date de publication et mention de la délivrance du brevet: **04.03.1998**
(21) Numéro de dépôt: 94420277.9
(22) Date de dépôt: 19.10.1994
(51) Int. Cl.: A61M 39/24

(54) **Dispositif pour l'injection médicale de liquides**
Infusionsvorrichtung
Medical injection device

(30) Priorité: 19.10.1993 FR 9312590
(43) Date de publication de la demande: 19.04.1995
(73) Titulaire: Medex, 74940 Annecy le Vieux (FR)
(72) Inventeur: Lacroix, Jean-Pierre, F-74940 Annecy le Vieux (FR)
(74) Mandataire: Poncet, Jean-François

(56) Documents cités:
- FR-A- 2 119 367
- GB-A- 2 000 685
- US-A- 2 538 662
- US-A- 4 114 617
- US-A- 4 573 974
- US-A- 5 025 829

## Description

La présente invention concerne les dispositifs à usage médical permettant l'injection de liquides dans le corps humain.

Les injections de liquides sont généralement effectuées au moyen d'un conduit d'injection tel qu'un cathelon, un cathéter, une aiguille hypodermique ou intraveineuse. Ce conduit d'injection, destiné à pénétrer dans le corps humain, est raccordé à une seringue ou à une canalisation d'injection de liquide pour injecter le liquide provenant d'un réservoir.

On connaît notamment des dispositifs d'injection permettant l'injection sélective de plusieurs liquides à partir de plusieurs sources de liquides. Ces dispositifs sont notamment décrits dans les documents US-A-4 573 974, US-A-5 025 829, US-A-4 114 617, FR-A-2 119 367.

Dans ces dispositifs, plusieurs canalisations comportant des valves antiretour conduisent respectivement le liquide depuis l'une des sources de liquide, et se rejoignent dans un raccord en Y prolongé par une canalisation de sortie raccordée à un conduit d'injection. Les canalisations conduisent les liquides unidirectionnellement depuis la sortie des sources d'alimentation en liquide jusqu'à l'entrée du conduit d'injection. Les sources d'alimentation en liquide sont le plus souvent des récipients disposés en hauteur, l'écoulement du liquide se faisant par gravité. Le document US-A-5 025 829 décrit des sources d'alimentation en liquide sous pression, avec des pompes. Le plus souvent, des clapets de contrôle de flux sont insérés dans les canalisations de conduction de liquide, avec éventuellement des chambres de goutte-à-goutte. Certains documents tels que le US-A-5 025 829, le US-A-4 114 617 et le FR-A-2 119 367 décrivent des vannes antiretour comportant un seuil par lequel la conduction de la valve nécessite une pression plus élevée à l'entrée qu'en sortie.

Le but poursuivi dans ces documents est de contrôler les écoulements respectifs de liquide provenant des sources d'alimentation en liquide respectives. Les canalisations se raccordent directement aux sources d'alimentation en liquide, et il n'est pas prévu de moyens particuliers pour déconnecter une partie des canalisations de conduction de liquide.

Les documents US-A-2 538 662 et GB-A-2 000 685 décrivent des éléments de canalisation de conduction de liquide comportant une valve antiretour. Dans les deux cas, la valve antiretour nécessite un flux inverse de liquide pour assurer la fermeture, de sorte qu'une contamination rétrograde est possible.

Dans tous ces dispositifs, aucun moyen n'est prévu pour permettre, dans des conditions de sécurité satisfaisantes, une utilisation du même dispositif pour plusieurs patients successifs.

On sait que les fluides présents à l'intérieur du corps humain ont tendance à pénétrer également dans la seringue ou dans la canalisation, en l'absence de débit. Cette pénétration est d'autant plus accentuée lorsque les injections se poursuivent sur une durée longue, ou sont répétées au cours de cette durée, sans que le dispositif d'injection soit retiré.

Ainsi, la pénétration des fluides du corps humain à l'intérieur des canalisations tend à contaminer l'ensemble du dispositif d'injection de liquides.

Le développement des maladies contagieuses, notamment du Sida, conduit ainsi à jeter la totalité du dispositif d'injection de liquides à la fin du traitement de chaque patient, pour éviter qu'un tel dispositif contaminé puisse lui-même contaminer ultérieurement un autre patient qui n'est pas atteint de la maladie. Il en résulte une diminution sensible de la durée d'usage des dispositifs d'injection de liquides, et une augmentation correspondante du coût de diagnostic ou de traitement des maladies.

Partant de l'état de la technique selon FR-A-2 119 367, le problème proposé par la présente invention est de concevoir une nouvelle structure de dispositif à usage médical pour l'injection de liquides qui permette d'éviter de jeter après traitement d'un patient la totalité du dispositif d'injection, tout en supprimant tout risque de contamination entre les patients successifs. On cherche ainsi à réutiliser le même dispositif de façon optimale pour plusieurs patients, pour minimiser les coûts de traitement.

L'efficacité anti-contamination du dispositif doit être assurée à la fois en autorisant des forts débits d'injection, c'est-à-dire des débits de l'ordre de 10 ml/s ou plus, et en s'opposant efficacement à toute transmission de germes infectieux, même de petite taille, dans les conditions habituelles d'utilisation, surtout en l'absence de débit.

De préférence, l'efficacité anti-contamination du dispositif doit pouvoir être contrôlée aisément pendant l'utilisation, afin de détecter le plus tôt possible les éventuels défauts et les éventuels risques de contamination entre les patients successifs.

Un autre objet de l'invention est de faciliter l'utilisation du dispositif d'injection, en évitant la sortie de fluides lors des déconnexions, notamment dans les dispositifs à source de liquide sous pression tels que les dispositifs utilisés en radiologie.

Le dispositif de l'invention doit également être compatible avec un ensemble d'injection susceptible d'être rempli plusieurs fois.

Pour atteindre ces objets ainsi que d'autres, l'invention prévoit un dispositif à usage médical pour l'injection de liquides selon la revendication 1

Le raccord amovible constitue lui-même un élément jetable, qui empêche toutes contaminations rétrogrades, c'est-à-dire tous passages de fluides, de bactéries ou de virus depuis le corps du patient jusqu'à l'embout d'entrée permettant son raccordement à une canalisation de sortie du dispositif d'alimentation en liquide. Ainsi, on s'assure que le dispositif d'alimentation en liquide avec sa canalisation de sortie n'est lui-même pas contaminé, et qu'il peut servir pour plusieurs patients successifs, à condition d'adapter à chaque fois un raccord amovible neuf ou décontaminé.

De préférence, les parties tubulaires amont et aval sont réalisées en un matériau transparent, laissant apparaître l'aspect du liquide qui les traverse. On peut ainsi, par un simple contrôle visuel, s'assurer de l'absence de contamination en amont de la valve lorsqu'une contamination est visible en aval de la valve, par exemple lors d'une coloration rouge ou rosée en présence de sang.

La partie tubulaire amont est destinée à contenir un liquide sous pression. Le liquide sous pression tend naturellement à repousser les parois des parties tubulaires, qui peuvent alors contenir du liquide en excès. Lors de la déconnexion du raccord amovible, l'élasticité naturelle des parois des parties tubulaires chasse l'excès de liquide, qui tend à s'écouler vers l'extérieur. Il en résulte un échappement d'une ou plusieurs gouttes de liquide.

Pour éviter cela, on peut soit utiliser une partie tubulaire amont rigide, soit prévoir qu'une telle partie tubulaire amont est la plus courte possible, par exemple d'une longueur comprise entre 1 et 4 centimètres environ.

D'autres objets, caractéristiques et avantages de la présente invention ressortiront de la description suivante de modes de réalisation particuliers, faite en relation avec les figures jointes, parmi lesquelles:
- la figure 1 est une vue schématique générale d'un dispositif d'injection de liquides à usage médical selon l'invention ;
- la figure 2 est une vue de face d'un raccord amovible selon un mode de réalisation de l'invention ;
- la figure 3 est une vue en coupe longitudinale d'un premier mode de réalisation de valve utilisable pour constituer le raccord de la figure 2 ;
- la figure 4 est une vue en coupe longitudinale d'une première pièce formant la moitié amont du corps de valve de la figure 3 ;
- la figure 5 est une vue en bout du côté aval de la pièce de la figure 4 ;
- la figure 6 est une coupe longitudinale d'une seconde pièce formant la moitié aval du corps de valve de la figure 3 ;
- la figure 7 est une vue du côté amont de la pièce de la figure 6 ;
- la figure 8 est une coupe longitudinale d'une valve antiretour à seuil selon un second mode de réalisation de la présente invention ;
- la figure 9 est une coupe longitudinale de la pièce centrale du corps de valve de la figure 8 ;
- la figure 10 est une vue en bout du côté aval de la pièce centrale de la figure 9 ; et
- la figure 11 est une vue en bout du côté amont de la pièce centrale de la figure 9.

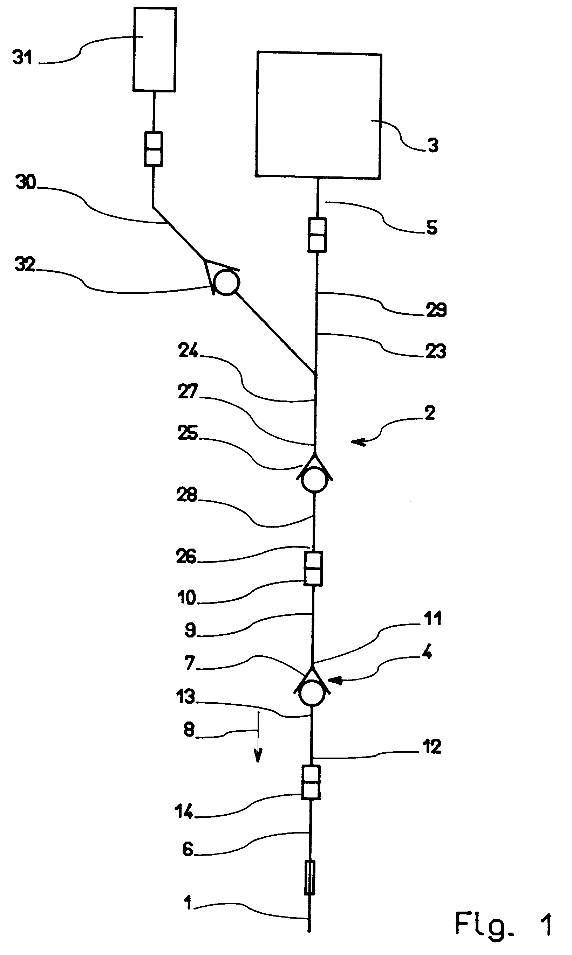

Comme le représente la figure 1, un dispositif à usage médical pour l'injection de liquides comprend généralement un conduit d'injection 1 tel qu'un cathelon, un cathéter, une aiguille hypodermique ou intraveineuse, raccordé par des moyens de canalisations de conduction de liquides 2 à un dispositif d'alimentation en liquide 3. Le dispositif d'alimentation en liquide 3 est adapté pour forcer le liquide sous pression dans les moyens de canalisations de conduction de liquides 2 et le conduit d'injection 1, pour injecter le liquide dans le corps d'un patient.

Selon l'invention, le dispositif d'injection de liquides comprend un raccord amovible 4, inséré dans les moyens de canalisations de conduction de liquides 2 entre la canalisation de sortie 5 du dispositif d'alimentation en liquide 3 et l'entrée 6 du conduit d'injection 1.

Le raccord amovible 4 comprend une valve antiretour à seuil 7, adaptée pour interdire le flux de liquide de sa sortie 13 vers son entrée 11, et adaptée pour autoriser le flux de liquide de son entrée 11 vers sa sortie 13 comme illustré par la flèche 8 lorsque la pression de liquide à son entrée excède la pression en sortie selon une valeur supérieure ou égale à un seuil de déclenchement déterminé S.

Le raccord amovible 4 comprend en outre une partie tubulaire amont 9, dont une première extrémité est munie d'un embout d'entrée 10 adapté pour permettre son raccordement déconnectable à une canalisation de sortie 5 du dispositif d'alimentation en liquide 3, et dont la seconde extrémité est raccordée à l'entrée 11 de la valve antiretour à seuil 7. Un simple tube amont, de préférence à section intérieure généralement constante sur toute sa longueur, relie les première et seconde extrémités de partie tubulaire amont 9.

Le raccord amovible 4 comprend également une partie tubulaire aval 12, dont une première extrémité est raccordée à la sortie 13 de la valve antiretour à seuil 7, et dont la seconde extrémité est munie d'un embout de sortie 14 adapté pour un raccordement déconnectable direct à l'entrée du conduit d'injection 1. Un simple tube aval, de préférence à section intérieure généralement constante sur toute sa longueur, relie les première et seconde extrémités de partie tubulaire aval 12.

L'embout d'entrée 10 peut être raccordé directement au dispositif d'alimentation en liquide 3, ou indirectement par la canalisation de sortie 5.

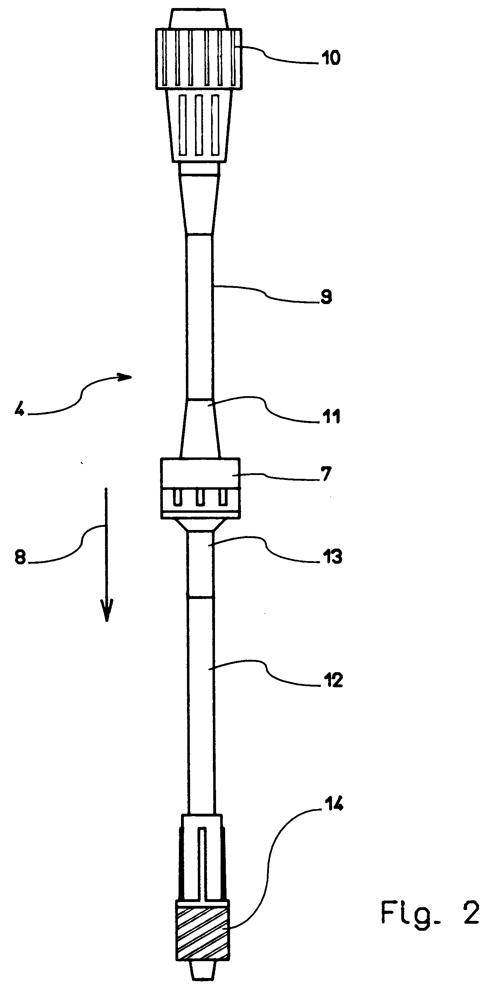

Comme illustré sur la figure 2, le raccord amovible 4 selon l'invention peut comprendre des embouts d'entrée 10 et de sortie 14 conformés et adaptés pour se raccorder de façon amovible à diverses canalisations dont les extrémités comprennent des embouts similaires. De préférence, il convient d'utiliser des embouts mâles et des embouts femelles de telle façon que l'on ne puisse pas inverser l'entrée et la sortie du raccord amovible 4, afin de respecter le sens de propagation 8 du liquide. Dans l'exemple illustré sur la figure, l'embout d'entrée 10 est un embout femelle, tandis que l'embout de sortie 14 est en embout mâle. Ainsi, l'embout de sortie 14 s'adapte à une entrée 6 de conduit d'injection munie d'un embout femelle correspondant, et l'embout d'entrée 10 s'adapte à un embout mâle prévu en sortie du dispositif d'alimentation en liquide 3 ou en sortie de la canalisation de sortie 5 qui raccorde le dispositif d'alimentation en liquide 3 et le raccord amovible 4. On pourra avantageusement utiliser des embouts répondant aux normes internationales en usage dans le domaine médical.

La partie tubulaire amont 9 et la partie tubulaire aval 12 sont de préférence réalisées en un matériau transparent, laissant apparaître l'aspect du liquide qui les traverse.

Pour assurer une liberté de déplacement et d'orientation du conduit d'injection 1, on réalise au moins la partie tubulaire aval 12 en un matériau lui conférant une flexibilité adaptée. Sa longueur peut avantageusement être comprise entre 10 et 30 centimètres. La partie tubulaire amont 9 peut également être flexible, pour augmenter les possibilités de déplacement et d'orientation du conduit d'injection 1.

De préférence, la partie tubulaire amont 9 a une longueur réduite, par exemple comprise entre 1 et 4 centimètres environ. Dans ce cas, cette partie tubulaire amont peut être réalisée en un matériau relativement déformable, de sorte qu'elle peut être flexible. La déformation de la paroi de partie tubulaire amont 9 sous l'effet de la pression du liquide qu'elle contient provoque une légère augmentation de sa capacité. Lors d'une déconnexion ultérieure de l'embout d'entrée 10, la paroi de partie tubulaire amont 9 tend à reprendre sa forme, en chassant l'excès de liquide qu'elle contient et qui s'écoule à l'extérieur. Pour éviter ou limiter un tel écoulement, la partie tubulaire amont 9 doit être courte, comme indiqué ci-dessus. En alternative, on peut utiliser une partie tubulaire amont 9 réalisée en un matériau rigide.

Le seuil de déclenchement S de la valve antiretour à seuil 7 est avantageusement compris entre 50 et 400 hecto Pascal. Grâce à un tel seuil de déclenchement, on s'assure qu'aucun fluide, aucune bactérie, ni aucun virus ne peut progresser depuis la partie tubulaire aval 12 jusque dans la partie tubulaire amont 9, même lorsque le dispositif d'alimentation en liquide 3 est au repos ou lorsque la partie tubulaire amont 9 est à une pression plus faible ou égale à la pression dans la partie tubulaire aval 12. Le passage dans la valve antiretour à seuil 7 ne se fait que lorsque la pression dans la partie tubulaire amont 9 est nettement supérieure à la pression dans la partie tubulaire aval 12, de sorte que le sens de progression du liquide dans la valve 7 est nécessairement conforme au sens indiqué par la flèche 8, de l'amont vers l'aval.

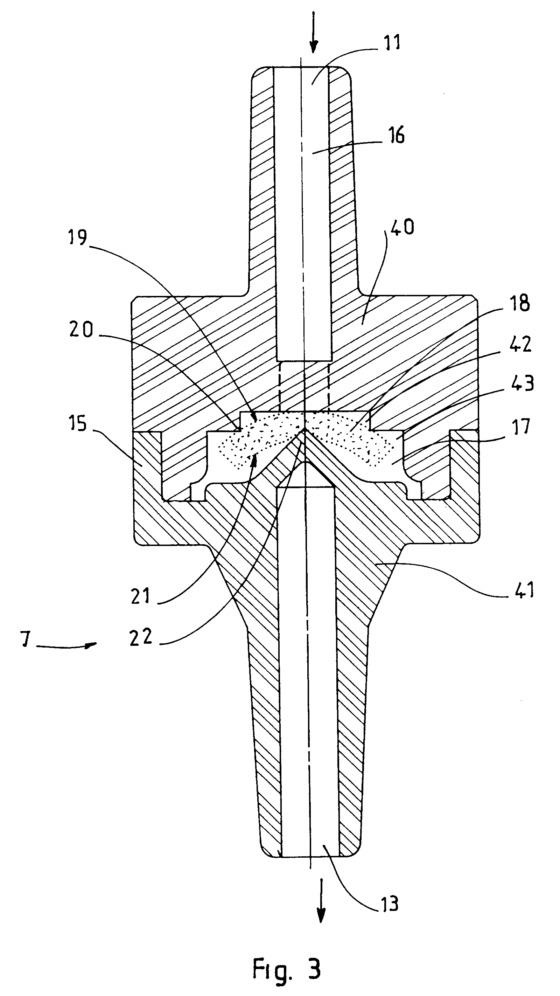

La figure 3 illustre un premier mode de réalisation de la valve antiretour à seuil 7, entièrement réalisée en matière plastique. La valve telle que représentée comprend un corps de valve 15 en deux parties 40 et 41 percées d'un canal traversant 16 dont la partie centrale forme une chambre d'obturation 17. La chambre d'obturation 17 comprend un siège intermédiaire 20 à arête vive formé à la jonction d'une portion amont 42 de chambre d'obturation à section plus réduite et d'une portion aval 43 de chambre d'obturation à section plus large. La chambre d'obturation 17 contient un disque obturateur 18 transversal élastiquement flexible dont le pourtour de la face amont 19 est en appui sur le siège 20 à arête vive du corps de valve 15 et dont la face aval 21 est contrainte axialement vers l'amont par un pointeau central 22 du corps de valve 15. Par déformation élastique sous l'action du pointeau 22, le disque obturateur 18 est plaqué contre le siège 20. On comprend que le disque obturateur 18 interdit le passage de fluide depuis la sortie 13 vers l'entrée 11, car toute surpression de fluide dans la sortie 13 plaque le disque obturateur 18 contre le siège 20. Par contre, le disque obturateur 18 autorise le passage de fluide depuis l'entrée 11 vers la sortie 13 lorsque la surpression à l'entrée 11 est suffisante pour fléchir élastiquement le pourtour du disque obturateur 18 et pour l'écarter légèrement du siège 20.

Le réglage du seuil de déclenchement S est réalisé par le choix approprié de l'épaisseur et de la dureté du disque obturateur 18.

Dans le mode de réalisation illustré sur la figure 3, le corps de valve 15 est formé de l'assemblage axial de deux pièces, une pièce 40 formant la première moitié amont du corps de valve, une pièce 41 formant la seconde moitié aval du corps de valve. Les deux pièces 40 et 41 comportent des nervures annulaires, comme représenté, conformées pour s'emboîter les unes dans les autres et réaliser à l'interface la chambre d'obturation 17. L'assemblage des pièces 40 et 41 peut, par exemple, être effectué par soudure à ultrasons, ou par tout autre moyen.

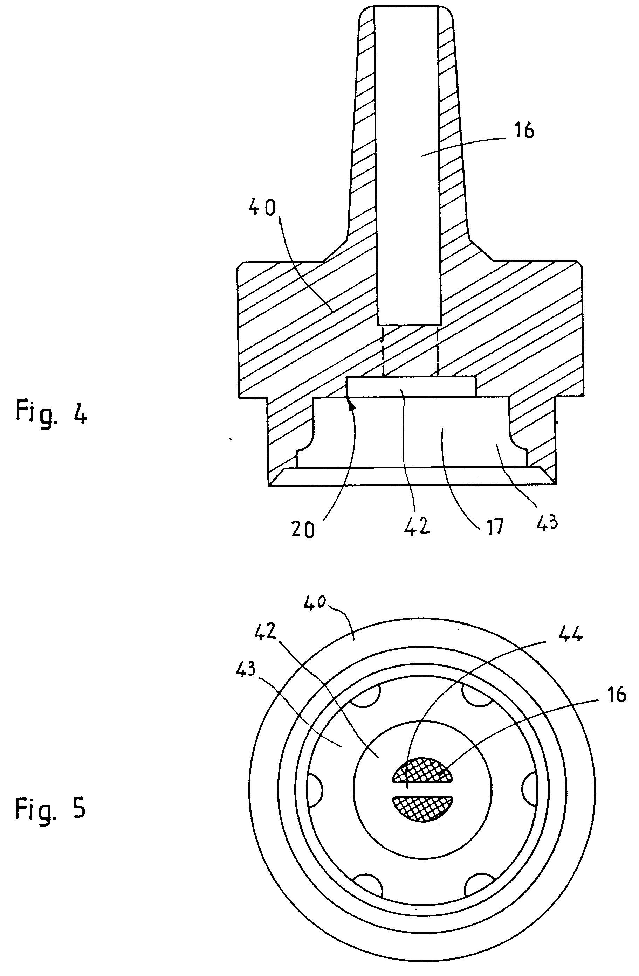

Les figures 4 et 5 illustrent la pièce 40 formant la moitié amont du corps de valve. Le canal traversant 16 comprend, à l'entrée de la chambre d'obturation 17, un pont diamétral 44 destiné à coopérer avec le disque obturateur 18. Le fluide peut passer librement de part et d'autre du pont diamétral 44.

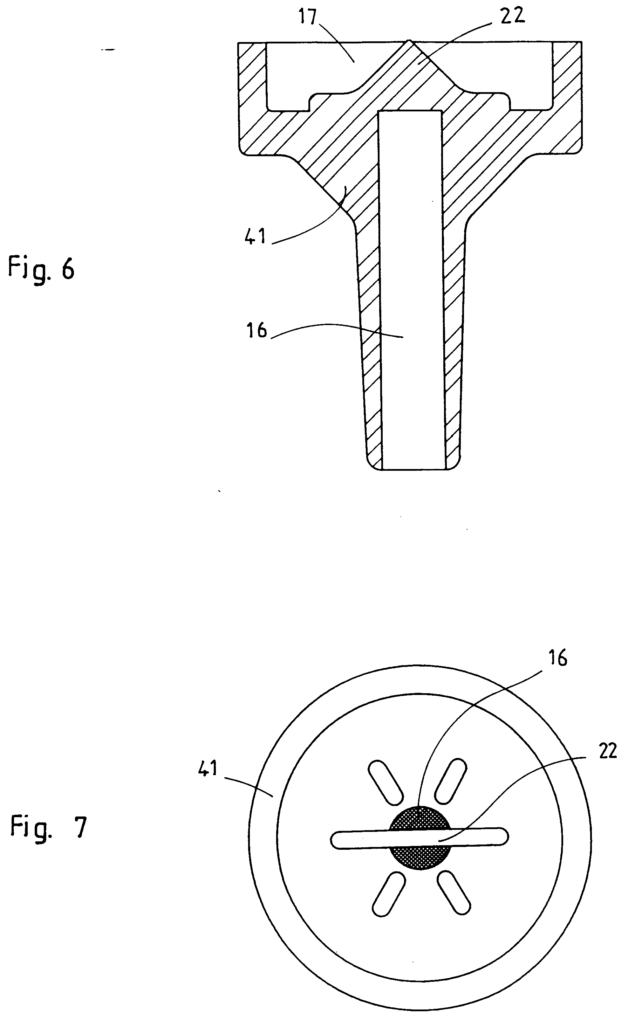

Les figures 6 et 7 illustrent la pièce 41 constituant la moitié aval du corps de valve. Le pointeau 22 est une nervure diamétrale généralement plate à profil longitudinal triangulaire, comme représenté. Les liquides peuvent passer de part et d'autre de la nervure formant le pointeau 22, depuis la chambre d'obturation 17 jusque dans le canal traversant 16.

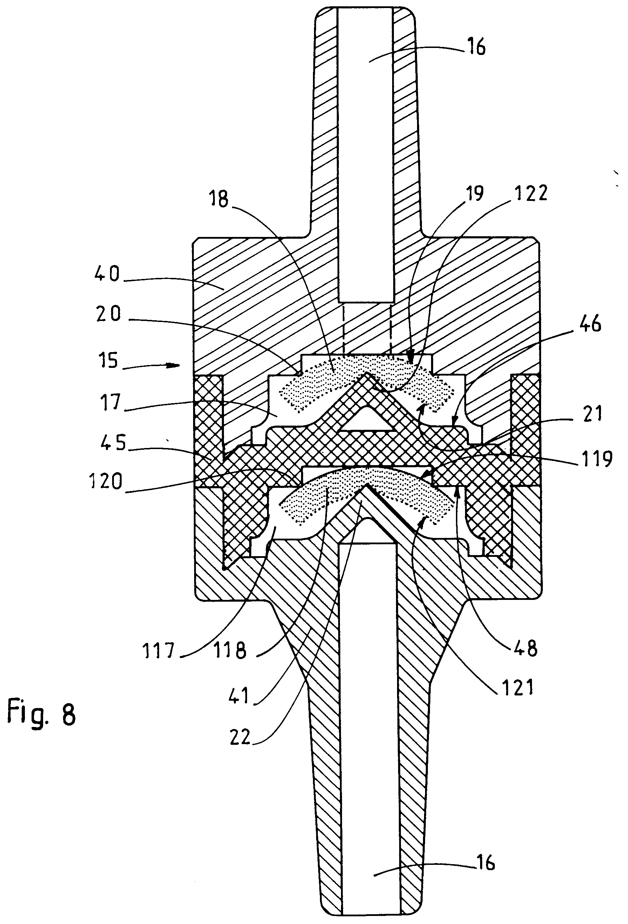

La figure 8 illustre, en coupe longitudinale, un second mode de réalisation préféré d'une valve antiretour à seuil selon la présente invention.

Pour assurer une fonction de sécurité améliorée, permettant de réduire encore les risques de contamination, on a constaté la nécessité d'utiliser une valve antiretour à seuil présentant un risque de passage inférieur à 10⁻⁵. Ce risque de passage est mesuré selon le test suivant : en aval de la valve antiretour, on introduit une solution aqueuse d'un produit dont les molécules sont de faible dimension, par exemple le produit ³H-2 désoxy-D-glucose, ou le produit interleukine 6rH. Du côté amont, on introduit une solution aqueuse dépourvue du produit à molécule de faible dimension. Le dispositif est maintenu dans cet état pendant une durée déterminée, par exemple 3 heures, à l'issue de laquelle on mesure la quantité de molécules de faible dimension qui ont pu passer du côté amont de la valve antiretour.

Il apparaît difficile de garantir, pour un lot de valves antiretour produites en grande série, un risque de passage inférieur à 10⁻⁵ avec une valve antiretour à seuil selon le mode de réalisation de la figure 3.

Par contre, le mode de réalisation de la figure 8 permet de réduire le risque de passage à une valeur inférieure à 10⁻⁵, procurant ainsi une sécurité satisfaisante pour les applications habituelles d'injections médicales.

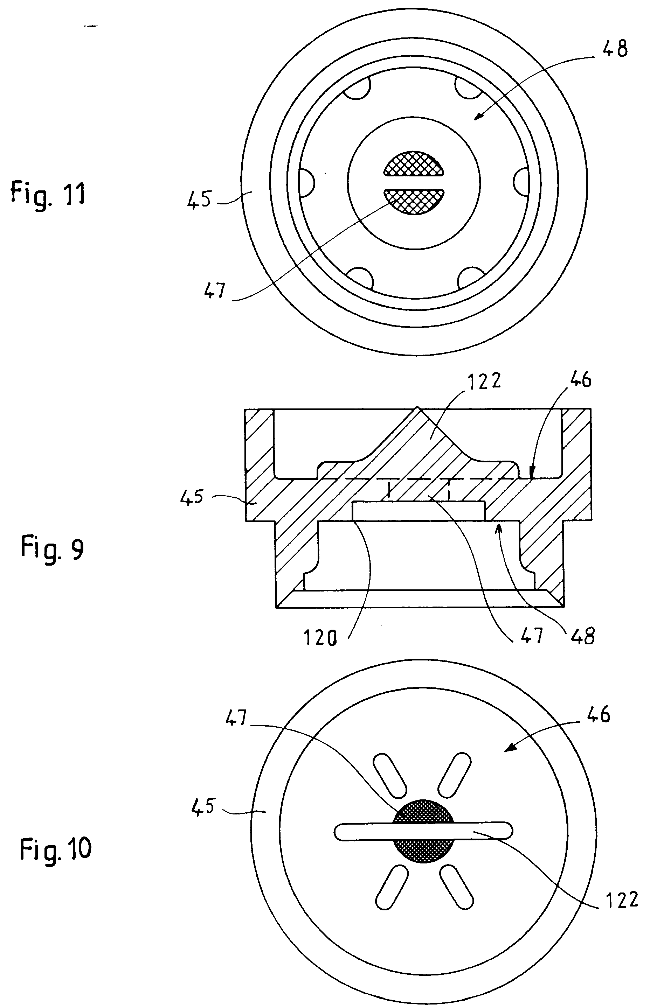

Dans ce mode de réalisation, le corps de valve 15 est réalisé par assemblage axial de trois parties 40, 41 et 45. La partie 40 peut avantageusement être identique à la pièce amont 40 du mode de réalisation de la figure 3. De même, la partie 41 peut avantageusement être identique à la pièce aval 41 du mode de réalisation de la figure 3. La partie 45 est une pièce intermédiaire 45 insérée entre la pièce amont 40 et la pièce aval 41. La pièce intermédiaire 45 comporte une face amont 46, également illustrée sur la figure 10, présentant la même forme que la face amont de la pièce aval 41, c'est-à-dire avec un pointeau central 122 similaire du pointeau 22, en forme de nervure diamétrale, et avec des passages latéraux 47 pour le passage de liquide de part et d'autre de la nervure formant le pointeau 122. De même, la pièce intermédiaire comporte une face aval 48, également représentée sur la figure 11, conformée comme la face aval de la pièce amont 40, c'est-à-dire avec un siège intermédiaire 120 similaire du siège 20 de la pièce amont 40. Ainsi, une première chambre d'obturation 17 est formée entre la pièce amont 40 et la pièce intermédiaire 45, et une seconde chambre d'obturation identique 117 est formée entre la pièce intermédiaire 45 et la pièce aval 41.

La première chambre d'obturation 17 contient le premier disque obturateur 18, dont le pourtour de la face amont 19 est en appui sur le premier siège intermédiaire 20 du corps de valve 15, et dont la face aval 21 est contrainte axialement vers l'amont par le pointeau central 122 de la pièce intermédiaire 45. De même, la seconde chambre d'obturation 117 contient un second disque obturateur 118 transversal élastiquement flexible dont le pourtour de la face amont 119 est en appui sur le siège intermédiaire 120 de la pièce intermédiaire 45 et dont la face aval 121 est contrainte axialement vers l'amont par le pointeau central 22 de la pièce aval 41 du corps de valve 15. Les disques obturateurs 18 et 118 réalisent une double obturation.

On comprend que la valve antiretour à seuil 7, la partie tubulaire amont 9, la partie tubulaire aval 12 et les embouts 10 et 14 peuvent être réalisés en matières plastiques moulées, les matières étant choisies pour être conformes aux normes d'usage médical, pour être compatibles avec les produits à injecter et pour être stérilisables par les méthodes usuelles.

Dans le mode de réalisation représenté sur la figure 1, le dispositif d'injection de liquides comprend, en amont du raccord amovible 4, une canalisation intermédiaire d'alimentation 23 comportant une branche de sortie 24 munie d'une seconde valve antiretour 25 à seuil, interdisant la remontée de liquide de sa sortie 26 vers son entrée 27, et autorisant le passage du liquide vers l'aval lorsque la pression à l'entrée 27 de la seconde valve antiretour 25 excède la pression à sa sortie 26 selon un second seuil de déclenchement déterminé S2.

La branche de sortie 24 comporte avantageusement, en aval de la seconde valve antiretour 25, une canalisation 28 courte, de longueur inférieure à deux centimètres environ. On évite ainsi l'écoulement d'excès de liquide lors de la déconnexion de l'embout d'entrée 10.

La canalisation intermédiaire d'alimentation 23 comprend en outre une branche d'entrée 29 adaptée pour relier l'entrée de la branche de sortie 24 à la canalisation de sortie 5 d'un dispositif d'alimentation en liquide 3 muni de moyens de génération de surpression et de dépression.

La canalisation intermédiaire d'alimentation 23 comprend également une branche de remplissage 30 adaptée pour relier l'entrée de la branche de sortie 24 à un réservoir de liquide 31, la branche de remplissage 30 étant munie d'une troisième valve antiretour 32 à troisième seuil de déclenchement S3 faible ou nul.

Lors d'une étape de remplissage, le dispositif d'alimentation en liquide 3 génère une dépression interne, provoquant un flux de liquide depuis le réservoir 31, à travers la troisième valve antiretour 32 et la branche de remplissage 30, et par la branche d'entrée 29 jusque dans le dispositif d'alimentation en liquide 3.

Lors d'une étape d'injection, le dispositif d'alimentation en liquide 3 repousse sous pression le liquide par la branche d'entrée 29, la branche de sortie 24, la seconde valve 25, le raccord amovible 4 et le conduit d'injection 1, lorsque la pression produite par le dispositif d'alimentation en liquide 3 est suffisante pour forcer le liquide à travers les deux valves antiretour successives 25 et 7.

Lors d'une déconnexion du raccord amovible 4, la seconde valve antiretour 25 empêche le flux de liquide depuis le réservoir 31 vers la sortie 26, à condition de prévoir que le second seuil S2 de la seconde valve 25 est supérieur à la pression de liquide résultant de la position surélevée du réservoir 31.

Dans les étapes au cours desquelles le dispositif d'alimentation en liquides 3 est au repos, la pression de liquide à l'intérieur de la partie tubulaire amont 9 peut être inférieure à la pression dans la partie tubulaire aval 12, mais la valve antiretour 7 interdit toute progression de liquide, de bactéries ou de virus depuis l'aval vers l'amont, et empêche la contamination de la partie tubulaire amont 9, de l'embout d'entrée 10 et de tous les éléments qui se trouvent en amont. En fin d'usage, la déconnexion de l'embout d'entrée 10 suffit à isoler le raccord amovible 4 et le conduit d'injection 1 par rapport aux autres éléments du dispositif qui ne sont pas contaminés. Le conduit d'injection 1 et le raccord amovible 4, qui sont éventuellement contaminés, sont ensuite jetés, ou traités par recyclage ou décontamination.

De préférence, le second seuil de déclenchement S2 de la seconde valve antiretour à seuil 25 est inférieur au premier seuil de déclenchement S de la première valve antiretour 7 du raccord amovible 4.

La présente invention n'est pas limitée aux modes de réalisation qui ont été explicitement décrits, mais elle en inclut les diverses variantes et généralisations contenues dans le domaine des revendications ci-après.

## Revendications

1. Dispositif à usage médical pour l'injection de liquides, comprenant des moyens de canalisations de conduction de liquides et un moyen de valve antiretour à seuil (7) pour conduire unidirectionnellement les liquides entre la sortie (5) d'un dispositif d'alimentation en liquide (3) sous pression et l'entrée (6) d'un conduit d'injection (1) tel que cathelon, cathéter, aiguille hypodermique ou intraveineuse, dispositif caractérisé en ce qu'il comprend :
- un raccord amovible (4) interchangeable formé de :
- un moyen de valve antiretour à seuil (7), adapté pour interdire le flux de liquide de sa sortie (13) vers son entrée (11), et pour autoriser le flux de liquide de son entrée (11) vers sa sortie (13) lorsque la pression de liquide à son entrée (11) excède la pression en sortie (13) selon une valeur supérieure ou égale à un seuil de déclenchement déterminé (S),
- une partie tubulaire amont (9), avec une première extrémité munie d'un embout d'entrée (10) femelle adapté pour un raccordement déconnectable à une canalisation de sortie (5) du dispositif d'alimentation en liquide (3), avec une seconde extrémité raccordée à l'entrée (11) du moyen de valve antiretour à seuil (7), et avec un simple tube amont reliant l'embout d'entrée (10) et l'entrée (11) du moyen de valve antiretour à seuil (7),
- une partie tubulaire aval (12), avec une première extrémité raccordée à la sortie (13) du moyen de valve antiretour à seuil (7), avec une seconde extrémité munie d'un embout de sortie (14) adapté pour un raccordement déconnectable direct à l'entrée (6) du conduit d'injection (1), et avec un simple tube aval reliant la sortie (13) du moyen de valve antiretour à seuil (7) et l'embout de sortie (14).

2. Dispositif selon la revendication 1, caractérisé en ce que le moyen de valve antiretour à seuil (7) comprend une première chambre d'obturation (17) contenant un premier disque obturateur (18), et une seconde chambre d'obturation (117) contenant un second disque obturateur (118), les chambres d'obturation (17, 117) étant successives et les disques obturateurs (18, 118) réalisant une double obturation.

3. Dispositif selon la revendication 2, caractérisé en ce que le moyen de valve antiretour à seuil (7) comprend un corps de valve (15) réalisé par assemblage de trois parties (40, 41, 45) percées de canaux (16) de passage de liquides et formant lesdites deux chambres d'obturation successives (17, 117) contenant chacune un disque obturateur (18, 118) transversal élastiquement flexible dont le pourtour de la face amont (19, 119) est en appui sur un siège intermédiaire (20, 120) du corps de valve (15) et dont la face aval (21, 121) est contrainte axialement vers l'amont par un pointeau central (22, 122) du corps de valve (15).

4. Dispositif selon l'une quelconque des revendications 1 à 3, caractérisé en ce que les parties tubulaires amont (9) et aval (12) sont réalisées en un matériau transparent, laissant apparaître l'aspect du liquide qui les traverse.

5. Dispositif selon l'une quelconque des revendications 1 à 4, caractérisé en ce qu'au moins la partie tubulaire aval (12) est réalisée en un matériau lui conférant une flexibilité adaptée au déplacement et à l'orientation du conduit d' injection (1).

6. Dispositif selon la revendication 5, caractérisé en ce que la partie tubulaire amont (9) est également flexible.

7. Dispositif selon la revendication 6, caractérisé en ce que la partie tubulaire amont (9) a une longueur comprise entre 1 et 4 centimètres environ.

8. Dispositif selon l'une quelconque des revendications 1 à 5, caractérisé en ce que la partie tubulaire amont (9) est réalisée en un matériau rigide.

9. Dispositif selon l'une quelconque des revendications 1 à 8, caractérisé en ce que le seuil de déclenchement déterminé (S) est compris entre 50 et 400 hecto Pascal.

10. Dispositif selon l'une quelconque des revendications 1 à 9, caractérisé en ce qu'il comprend en outre une canalisation intermédiaire d'alimentation (23) comportant :
- une branche de sortie (24) munie d'une seconde valve antiretour (25) à seuil interdisant la remontée de liquide de sa sortie (26) vers son entrée (27) et autorisant le passage du liquide vers l'aval lorsque la pression à l'entrée (27) de la seconde valve antiretour (25) excède la pression à sa sortie (26) selon un second seuil de déclenchement déterminé (S2), la branche de sortie (24) comportant en aval de la seconde valve antiretour (25) une canalisation (28) courte de longueur inférieure à deux centimètres environ,
- une branche d'entrée (29), adaptée pour relier l'entrée de la branche de sortie (24) à un dispositif d'alimentation en liquide (3) muni de moyens de génération de surpression et de dépression,
- une branche de remplissage (30), adaptée pour relier l'entrée de la branche de sortie (24) à un réservoir de liquide (31), la branche de remplissage (30) étant munie d'une troisième valve antiretour (32) à seuil (S3) faible ou nul.

11. Dispositif selon la revendication 10, caractérisé en ce que le second seuil de déclenchement (S2) de seconde valve antiretour (25) est inférieur au premier seuil de déclenchement (S) de la valve antiretour (7) du raccord amovible (4).

## Claims

1. Device for medical use for injecting liquids, including liquid conduction tube means and a threshold check valve means (7) to transport liquids unidirectionally between the outlet (5) of a liquid supplying device (3) under pressure and the inlet (6) of an injection conduit (1) such as a cathelon, a catheter, a hypodermic or intravenous needle, device characterized in that it comprises :
- a removable and interchangeable connecting piece (4) composed of :
- a threshold check valve (7) adapted to prevent the flow of liquid from its outlet (13) to its inlet (11) and to authorize the flow of liquid from its inlet (11) to its outlet (13) when the liquid pressure at its inlet (11) exceeds the outlet pressure (13) according to a value which is greater than or equal to a determined activation threshold (S),
- an upstream tubular part (9), with a first end provided with a inlet female end piece (10) adapted for a deconnectable connection to an outlet tube (5) of the liquid supplying device (3), with a second end connected to the inlet (11) of the threshold check valve (7), and with a simple upstream tube connecting the inlet end piece (10) to the inlet (11) of the threshold check valve means (7).
- a downstream tubular part (12), with a first end connected to the outlet (13) of the threshold check valve means (7), with a second end provided with an outlet end piece (14) adapted for a direct deconnectable connection to the inlet (6) of the injection conduit (1), and with a simple downstream tube connecting the outlet (13) of the threshold check valve means (7) to the outlet end piece (14).

2. Device according to Claim 1, characterized in that the threshold check valve means (7) comprises a first stopping chamber (17) containing a first stopping disc (18), and a second stopping chamber (117) containing a second stopping disc (118), with the stopping chambers (17, 117) being successive and the stopping discs (18, 118) carring out a double stoppage.

3. Device according to Claim 2, characterized in that the threshold check valve (7) includes a valve body (15) made by assembling three parts (40, 41, 45) drilled with channels (16) for passing liquid and forming the said successive stopping chambers (17, 117) each containing a resiliently supple transverse stopping disc (18, 118) the upstream face periphery (19, 119) of which bears upon a intermediary seat (20, 120) of the valve body (15) and the downstream face (21, 121) of which is axially stressed upstream by a central needle (22, 122) in the valve body (15).

4. Device according to any one of Claims 1 to 3, characterized in that the upstream (9) and downstream (12) tubular parts are made out of a transparent material, thus displaying the appearance of the liquid passing through them.

5. Device according to any one of Claims 1 to 4 characterized in that at least the downstream tubular part (12) is made out of a material giving it a suppleness adapted to the movement and the orientation of the injection conduit (1).

6. Device according to Claim 5, characterized in that the upstream tubular part (9) is also supple.

7. Device according to Claim 6, characterized in that the length of the upstream tubular part (9) is between 1 and 4 centimeters approximately.

8. Device according to any one of Claims 1 to 5, characterized in that the upstream tubular part (9) is made out of a rigid material.

9. Device according to any one of Claims 1 to 8, characterized in that the determined activation threshold (S) is between 50 and 400 hectopascal.

10. Device according to any one of Claims 1 to 9, characterized in that it further comprises an intermediary feeding tube (23) including :
- an outlet branch (24) provided with a second threshold check valve (25) preventing liquid from going back up from its outlet (26) to its inlet (27) and authorizing the liquid to pass downstream when the pressure at the inlet (27) of the second check valve (25) exceeds the pressure at its outlet (26) according to a second determined activation threshold (S2), the oulet branch (24) comprising, downstream from the second check valve (25), a short tube (28) which is less than 2 centimeters long approximately,
- an inlet branch (29) adapted to connect the inlet of the outlet branch (24) up to a liquid supplying device (3) provided with overpressure and negative pressure generation means,
- a filling branch (30) adapted to connect the inlet of the outlet branch (24) up to a liquid tank (31), the filling branch (30) being equipped with a third weak or nil threshold (S3) check valve (32).

11. Device according to Claim 10, characterized in that the second activation threshold (S2) of the second check valve (25) is less than the first activation threshold (S) of the check valve (7) of the removable connection piece (4).

## Patentansprüche

1. Vorrichtung zum medizinischen Gebrauch für die Injektion von Flüssigkeiten, bestehend aus Leitungsmitteln zur Hindurchleitung von Flüssigkeiten und einem Rückschlag-Schwellenventilmittel (7) zum Hindurchleiten von Flüssigkeiten in einer Richtung zwischen dem Ausgang (5) einer Druckflüssigkeit-Versorgungsvorrichtung (3) und dem Eingang (6) einer Injektionsleitung (1), wie bspw. Kathelon, Katheter, subkutane oder intravenöse Nadel, wobei die Vorrichtung dadurch gekennzeichnet ist, daß sie besteht aus:
- einem lösbaren Anschluß (4), der austauschbar ausgebildet ist aus:
- einem Rückschlag-Schwellenventilmittel (7), welches zur Verhinderung des Flüssigkeitsflusses von seinem Ausgang (13) gegen seinen Eingang (11) angepaßt ist und zur Ermöglichung des Flüssigkeitsflusses von seinem Eingang (11) gegen seinen Ausgang (13), sobald der Flüssigkeitsdruck an seinem Eingang (11) den Druck am Ausgang (13) um einen Wert übersteigt, der größer oder gleich einer bestimmten Auslöseschwelle (S) ist,
- einem stromaufwärts verlaufenden rohrförmigen Teil (9) mit einem ersten Ende, das mit einem weiblichen Eingangsansatzstück (10) für einen trennbaren Anschluß an eine Ausgangsleitung (5) der Flüssigkeit-Versorgungsvorrichtung (3) versehen ist, mit einem zweiten Ende, das mit dem Eingang (11) des Rückschlag-Schwellenventilmittels (7) verbunden ist, und mit einer stromaufwärts angeordneten einfachen Röhre, welche das Eingangsansatzstück (10) und den Eingang (11) des Rückschlag-Schwellenventilmittels (7) miteinander verbindet,
- einem stromabwärts verlaufenden rohrförmigen Teil (12) mit einem ersten Ende, das mit dem Ausgang (13) des Rückschlag-Schwellenventilmittels (7) verbunden ist, mit einem zweiten Ende, das mit einem Ausgangsansatzstück (14) versehen ist, welches für einen trennbaren Anschluß direkt mit dem Eingang (6) der Injektionsleitung (1) angepaßt ist, und mit einer stromabwärts angeordneten einfachen Röhre, welche den Ausgang (13) des Rückschlag-Schwellenventilmittels (7) und das Ausgangsansatzstück (14) miteinander verbindet.

2. Vorrichtung nach Anspruch 1, dadurch gekennzeichnet, daß das Rückschlag-Schwellenventilmittel (7) eine erste Verschlußkammer (17) aufweist, die eine erste Verschlußscheibe (18) enthält, und eine zweite Verschlußkammer (117), die eine zweite Verschlußscheibe (118) enthält, wobei die Verschlußkammern (17, 117) aufeinander folgen und die Verschlußscheiben (18, 118) einen Doppelverschluß realisieren.

3. Vorrichtung nach Anspruch 2, dadurch gekennzeichnet, daß das Rückschlag-Schwellenventilmittel (7) einen Ventilkörper (15) aufweist, der durch den Zusammenbau von drei Teilen (40, 41, 45) realisiert ist, die mit Leitungen (16) für die Hindurchleitung von Flüssigkeiten durchbohrt sind und die beiden aufeinanderfolgenden Verschlußkammern (17, 117) bilden, die jeweils eine transversal elastisch flexible Verschlußscheibe (18, 118) enthalten, bei welcher der äußere Umfang der stromaufwärts befindlichen Stirnseite (19, 119) an einem Zwischensitz (20, 120) des Ventilkörpers (15) anliegt und deren stromabwärts befindliche Stirnseite (21, 121) axial in der Richtung stromaufwärts durch eine zentrale Injektornadel (22, 122) des Ventilkörpers (15) belastet ist.

4. Vorrichtung nach einem der Ansprüche 1 bis 3, dadurch gekennzeichnet, daß die stromaufwärts (9) und stromabwärts (12) verlaufenden rohrförmigen Teile mit einem transparenten Material realisiert sind, welches das Aussehen der sie durchquerenden Flüssigkeit erscheinen läßt.

5. Vorrichtung nach einem der Ansprüche 1 bis 4, dadurch gekennzeichnet, daß wenigstens der stromabwärts verlaufende rohrförmige Teil (12) aus einem Material realisiert ist, welches ihm eine Flexibilität verleiht, die der Verschiebung und der Orientierung der Injektionsleitung (1) angepaßt ist.

6. Vorrichtung nach Anspruch 5, dadurch gekennzeichnet, daß auch der stromaufwärts verlaufende rohrförmige Teil (9) flexibel ist.

7. Vorrichtung nach Anspruch 6, dadurch gekennzeichnet, daß der stromaufwärts verlaufende rohrförmige Teil (9) eine Länge aufweist, die etwa zwischen 1 und 4 cm beträgt.

8. Vorrichtung nach einem der Ansprüche 1 bis 5, dadurch gekennzeichnet, daß der stromaufwärts verlaufende rohrförmige Teil (9) aus einem unelastischen Material realisiert ist.

9. Vorrichtung nach einem der Ansprüche 1 bis 8, dadurch gekennzeichnet, daß die bestimmte Auslöseschwelle (S) mit zwischen 50 und 400 Hektopascal gebildet ist.

10. Vorrichtung nach einem der Ansprüche 1 bis 9, dadurch gekennzeichnet, daß sie weiterhin eine dazwischenliegende Versorgungsleitung (23) aufweist, bestehend aus:
- einer Ausgangszweigleitung (24), die mit einem zweiten Rückschlag-Schwellenventil (25) versehen ist, welches das Ansteigen der Flüssigkeit von ihrem Ausgang (26) gegen ihren Eingang (27) verhindert und den Durchfluß der Flüssigkeit in der Richtung stromabwärts erlaubt, sobald der Druck am Eingang (27) des zweiten Rückschlagventils (25) den Druck an seinem Ausgang (26) übersteigt gemäß einer zweiten bestimmten Auslöseschwelle (S2), wobei die Ausgangszweigleitung (24) stromabwärts von dem zweiten Rückschlagventil (25) eine kurze Leitung (28) mit einer Länge von weniger als etwa 2 cm aufweist,
- einer Eingangszweigleitung (29), die für eine Verbindung des Eingangs der Ausgangszweigleitung (24) mit einer Flüssigkeit-Versorgungsvorrichtung (3) angepaßt ist, die mit Mitteln zur Erzeugung eines Überdruckes und eines Unterdruckes versehen ist,
- einer Füllungszweigleitung (30), die zur Verbindung des Eingangs der Ausgangszweigleitung (24) mit einem Flüssigkeitsreservoir (31) angepaßt ist, wobei die Füllungszweigleitung (30) mit einem dritten Rückschlagventil (32) mit einer schwachen oder nicht vorhandenen Schwelle (S3) versehen ist.

11. Vorrichtung nach Anspruch 10, dadurch gekennzeichnet, daß die zweite Auslöseschwelle (S2) des zweiten Rückschlagventils (25) geringer ist als die erste Auslöseschwelle (S) des Rückschlagventils (7) des lösbaren Anschlusses (4).
